# FASCICULE DE BREVET EUROPEEN

(11) **EP 1 265 647 B1**
(45) Date de publication et mention de la délivrance du brevet: **10.12.2003**
(21) Numéro de dépôt: 00925383.2
(22) Date de dépôt: 04.05.2000
(51) Int. Cl.: A61L 11/00

(54) **PROCEDE ET DISPOSITIF DE BROYAGE ET DE DESINFECTION DES DECHETS MEDICAUX**
VERFAHREN UND VORRICHTUNG ZUR ZERKLEINERUNG UND DESINFEKTION VON MEDIZINISCHEN ABFÄLLEN
METHOD AND DEVICE FOR GRINDING AND DISINFECTING MEDICAL WASTE

(30) Priorité: 07.05.1999 FR 9905967
(43) Date de publication de la demande: 18.12.2002
(73) Titulaire: JMG Holding SARL, 13500 Martigues (FR)
(72) Inventeur: HENGL, Patrick, 31450 Issus (FR)
(74) Mandataire: Roman, Michel
(86) Numéro de dépôt international: FR0001199
(87) Numéro de publication internationale: WO00067808

(56) Documents cités:
- EP-A- 0 510 276
- EP-A- 0 763 390
- US-A- 4 578 185

## Description

La présente invention est du domaine du traitement des déchets notamment des déchets médicaux. L'invention se destine essentiellement au broyage et à la stérilisation des déchets afin d'éliminer tous les agents nocifs et propose un procédé et un dispositif à cet effet. L'intérêt d'un tel système est de décontaminer des déchets infectieux afin qu'ils puissent être assimilés par la chaîne de traitement des déchets comme des déchets ménagers classiques. Ce type de dispositif est particulièrement intéressant pour des professions médicales produisant peu de déchets infectieux et pour lesquelles il est difficile d'effectuer une collecte de déchets spécifique.

Le domaine du traitement des déchets médicaux utilisant le principe de broyage et stérilisation a été évoqué dans de nombreux brevets.

On peut citer par exemple le brevet FR 2 686 794 qui décrit une installation d'hygiénisation des déchets dans laquelle les déchets sont finement broyés puis les particules ainsi obtenues sont traitées au moyen de vapeur d'eau.

On connaît également le brevet FR 2 757 063 décrivant un procédé de stérilisation consistant à mouiller les matériaux, à les réduire en poudre puis à les chauffer pour les stériliser.

Dans ces deux brevets, les moyens de broyage sont situés en aval des moyens de stérilisation. Tout le dispositif d'alimentation et de broyage des déchets se trouve donc contaminé par le contact avec les déchets non encore stérilisés. Après le broyage, les déchets sont transférés vers le poste de stérilisation. Il est évident qu'il subsiste de nombreuses particules de déchets sur les parois du dispositif ainsi que sur les lames du broyeur. Le dispositif de broyage lui même devient ainsi une source de contamination importante vers l'extérieur.

Le brevet FR 2 715 851 a tenté de trouver une solution à ce problème en proposant une installation de stérilisation-broyage composée d'une cuve comportant un dispositif de broyage. Les déchets sont introduits dans la cuve. Les déchets sont alors soumis au broyage et à la vapeur. Les déchets sont ensuite évacués puis la cuve est refermée pour effectuer son nettoyage. A cet effet, la cuve est remplie d'eau puis on y injecte de la vapeur.

Même si elle permet de réduire la contamination des déchets résiduels du broyeur, cette installation ne permet pas leur élimination complète. En effet, cette installation ne permet pas de décoller et de nettoyer parfaitement la surface des lames du broyeur.

Le brevet EP 510 276 (WINFIELD IND) décrit une installation de traitement des déchets. Les moyens de désinfection des lames du broyeur comportent des buses (46b) et (46c) (figure 3 dudit brevet) disposées en vis-à-vis, de part et d'autre des lames. Les buses permettent de soumettre les lames du broyeur à un brouillard de vapeur mais il n'y aura par réellement d'action mécanique du fluide sur l'ensemble de la surface de chaque lame permettant de décoller les résidus.

Le brevet EP 763 390 (RIVERA MONTANES CARMELO) décrit un système de traitement de déchets hospitaliers. Les déchets sont introduits dans des sacs, broyés et introduits dans une enceinte au fond de laquelle est disposé un système de compactage des déchets.

L'une des étapes du procédé consiste à introduire de la vapeur (4) dans l'enceinte. Il n'est en aucun cas décrit que le flux de vapeur est directement dirigé sur les lames du broyeur. Ainsi, il n'y a aucune action mécanique du fluide sur les lames du broyeur permettant de décoller les résidus de déchets. Cette méthode de désinfection ne peut donc pas être totalement efficace.

Le brevet US 4 578 185 (WILSON JOSEPH H ET AL) décrit une installation de broyage de déchets. On observe sur la figure 3 dudit brevet que la trémie d'admission des déchets dans le broyeur est surplombée par une buse de projection d'un fluide désinfectant. Cette buse a pour rôle de soumettre les déchets, entrants dans le dispositif, au liquide désinfectant. Une fois encore, ces moyens de désinfection n'ont aucune action directe sur les lames du broyeur permettant d'éliminer et résidus et de désinfecter les lames.

La présente invention a pour objet de proposer un procédé et un dispositif de broyage et de désinfection des déchets permettant d'obtenir une parfaite décontamination des déchets et de maintenir le dispositif totalement propre et exempt de tous résidus de déchets.

Le procédé selon l'invention est caractérisé en ce qu'il comporte une étape consistant à projeter au moins un fluide directement sur les lames du broyeur, ledit fluide étant projeté dans une direction parallèle à la surface de chaque lame, depuis le centre de la lame vers l'extérieur afin d'éliminer et de décontaminer tous les résidus de déchets que lesdites lames comportent.

Le dispositif de broyage à couteaux rotatifs selon la présente invention comporte des moyens spécifiques de projection d'au moins un fluide sur les lames dudit broyeur, caractérisé en ce que le fluide de nettoyage des lames du broyeur est projeté au travers d'au moins un canal réalisé au niveau de chaque axe de rotation dudit broyeur.

Selon une autre caractéristique de l'invention, le fluide de nettoyage des lames du broyeur est projeté au travers d'orifices situés au niveau l'arbre de rotation dudit broyeur.

D'autres avantages et caractéristiques de l'invention apparaîtront à la lecture de la description ci-après de formes de réalisation de l'invention données à titre d'exemples non limitatifs et illustrés par les dessins joints dans lesquels:
- la figure 1 représente en coupe, en vue de face et en vue de coté les différents éléments du dispositif de broyage et de désinfection.
- la figure 2 représente en coupe, en vue de face et en vue de coté, l'assemblage des lames sur l'axe du broyeur.
- la figure 3 représente une lame du broyeur.

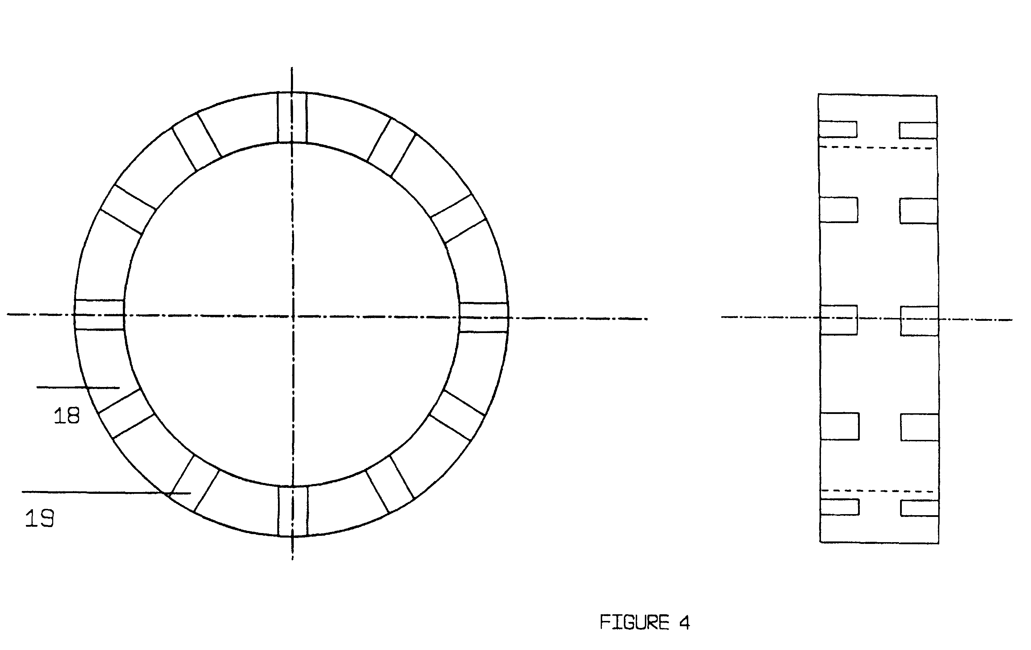

- la figure 4 représente l'une des entretoises séparant les lames du broyeur.
- la figure 5 représente une variante de réalisation du dispositif.

Le procédé selon l'invention consiste à introduire les déchets dans le dispositif, à les broyer finement puis à les chauffer à haute température. Les déchets ainsi stérilisés sont compactés puis évacués. Le procédé comporte également une phase de nettoyage du dispositif consistant à remplir le dispositif de broyage par un liquide bactéricide, à vidanger ce liquide puis à projeter au moins un fluide sur les lames du broyeur. Selon un mode particulier de réalisation de l'invention, le fluide est projeté selon une direction parallèle à la surface des lames du broyeur préférentiellement depuis le centre de la lame vers l'extérieur de manière à chasser les résidus par son action mécanique. Pour un nettoyage efficace, le fluide projeté peut être de la vapeur d'eau portée à une température supérieure à 130°C. Il est également possible de projeter un fluide tel que du liquide bactéricide ayant une action mécanique plus forte que celle de la vapeur. La pression de projection du fluide peut être variable, de l'ordre de quelques bars par exemple. Le liquide peut être projeté dans un premier temps puis suivi de la projection de vapeur. Cette projection a un double effet, l'action mécanique du liquide permet de décoller les résidus de déchets collés sur les lames du broyeur et la vapeur permet une désinfection totale par l'effet de chaleur. La projection de liquide suivi de la projection de vapeur peut être obtenue grâce à la phase de remplissage du dispositif de broyage durant laquelle une partie du liquide bactéricide vient se loger dans les orifices de projection de vapeur. En envoyant la vapeur sous pression, le liquide sera propulsé et viendra nettoyer la surface des lames.

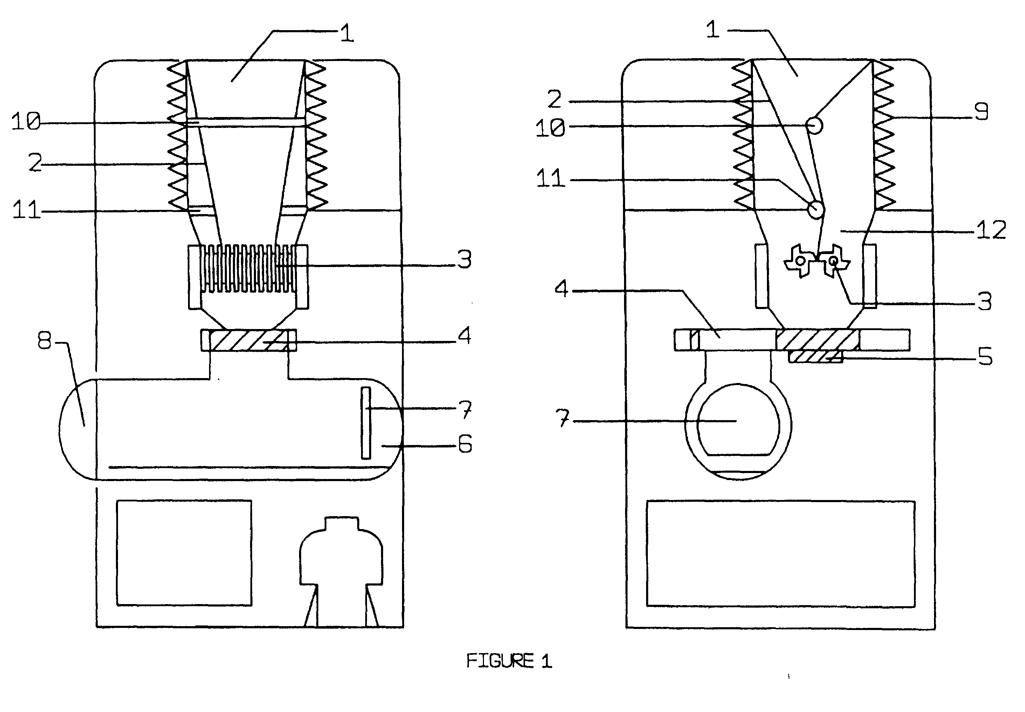

Comme on peut le voir sur la figure 1, le dispositif de broyage et de stérilisation selon l'invention est constitué:
- d'un orifice d'alimentation (1) des déchets,
- d'une gaine souple (2) enfilée sur l'orifice d'alimentation des déchets et dont l'extrémité supérieure est introduite dans l'orifice de manière à recouvrir la paroi interne dudit orifice,
- d'un dispositif de broyage (3) contenu dans une enceinte de broyage ( 12)
- d'une vanne à tiroir (4)
- d'un plateau de pesage (5 )
- d'un autoclave (6) de désinfection des déchets
- d'un plateau de pressage des déchets (7)
- d'un orifice d'extraction des déchets désinfectés.

Les déchets contaminés sont introduits dans le dispositif de broyage et de désinfection par l'orifice d'alimentation (1) situé sur la partie supérieure du dispositif. L'orifice d'alimentation (1) des déchets peut être de forme tubulaire sur laquelle la gaine souple est enfilée. Cette gaine (2) est constituée d'une membrane souple de forme tubulaire par exemple, enfilée sur la paroi externe (9) de l'orifice d'alimentation et dont l'extrémité supérieure est introduite dans la partie interne de l'orifice. L'orifice d'alimentation des déchets est pourvu d'un système de tension de la gaine constitué de deux axes parallèles et superposés (10) et (11) entre lesquels passe la gaine. L'extrémité interne de la gaine est engagée dans le dispositif de broyage. La gaine (2) recouvre donc la paroi interne de l'orifice d'alimentation ce qui évite tout contact direct des déchets contaminés sur l'orifice.

Le dispositif de broyage est du type à couteaux rotatifs. Selon un mode de réalisation de l'invention, le dispositif de broyage est constitué de deux arbres parallèles dans un plan horizontal portant chacun des lames sous forme de disques tranchants. Les deux arbres sont entraînés en rotation dans des sens opposés de manière à happer les déchets vers le bas.

La rotation du dispositif de broyage a pour effet de happer la gaine et d'entraîner en même temps les déchets. La gaine et les déchets sont donc broyés en même temps. Au fur et à mesure que la gaine est happée par le dispositif de broyage, celle-ci défile de l'orifice d'alimentation.

Les déchets et la gaine ainsi broyés se déposent dans une vanne à tiroir (4) au fond de laquelle est disposé un plateau de pesage. Lorsqu'une certaine quantité de déchets broyés est atteinte, le dispositif de broyage s'arrête et la vanne à tiroir translate horizontalement pour transférer par gravité les déchets vers l'autoclave. L'autoclave est situé sous le dispositif de broyage. La vanne à tiroir (4) permet dans une position de collecter les déchets broyés et dans l'autre position de les transférer vers l'autoclave (6). Les déchets ainsi transférés dans l'autoclave peuvent être stérilisés en les soumettant à une température de l'ordre de 135°C pendant environ 20 minutes.

Lorsque les déchets sont stérilisés, ceux-ci sont évacués par l'orifice d'évacuation (8) situé sur le coté du dispositif et fermé par une porte. L'autoclave comporte un plateau de pressage (7) mobile en translation horizontale et actionné par un vérin par exemple. Ce plateau a pour fonction de presser les déchets contre la porte de l'orifice d'évacuation afin de les essorer et de faciliter leur évacuation. L'ensemble des éléments de motorisation et de commande électrique du dispositif est disposé dans la partie inférieure du dispositif, sous l'autoclave.

Le dispositif selon l'invention prévoit un système spécifique de nettoyage et de décontamination du dispositif lui-même. En effet, en amont du dispositif de broyage, la gaine empêche tout contact des déchets avec les parois du dispositif mais entre la phase de broyage et la phase de stérilisation, les déchets peuvent contaminer certains éléments du dispositif. Les éléments présentant ce risque sont essentiellement l'enceinte de broyage et le dispositif de broyage.

L'enceinte de broyage pourvue de joints étanches au niveau de la vanne à tiroir pour permettre son remplissage par un liquide bactéricide. Cette solution bactéricide aura pour objectif de noyer le dispositif de broyage afin d'éliminer toute contamination. Le remplissage et la vidange de l'enceinte de broyage sont effectués par des moyens spécifiques non représentés, la pompe permettant le remplissage et la vidange pouvant par exemple être une pompe péristaltique.

L'invention prévoit également un moyen spécifique de nettoyage des lames du broyeur. A cet effet, le nettoyage des lames du broyeur est effectué en projetant au moins un fluide parallèlement à la surface des lames. Ce dispositif de projection de fluide peut par exemple être disposé au-dessus des lames.
Selon un mode préféré de réalisation de l'invention, la vapeur d'eau ou tout autre fluide est projeté au travers de canaux réalisés au niveau de chaque axe de rotation du broyeur. D'autre part les canaux de projection de fluide sont conçus de manière à permettre le stockage de liquide qui viendra s'introduire dans lesdits canaux durant la phase de remplissage de l'enceinte de broyage par la solution bactéricide. Ainsi, lorsque l'on projette la vapeur, les restes de liquide stockés dans les canaux sont dans un premier temps projetés avec un impact mécanique plus fort que celui provoqué par les projections de la vapeur d'eau. Lorsque les restes de liquide sont évacués, c'est la vapeur qui achève la décontamination par l'effet de chaleur

La projection du fluide est donc effectuée au travers de canaux parallèles à l'axe de rotation auxquels sont reliés des canaux radiaux débouchant vers l'extérieur de manière à projeter le fluide sur la surface de la lame. Ce sont ces mêmes canaux qui permettent le stockage du liquide.

Les modes de réalisations des canaux axiaux et radiaux sont nombreux et des variantes du mode de réalisation décrit ci-après sont facilement exécutables par l'homme de l'art.

Chaque axe de broyage est constitué d'un arbre (16) et de lames (17) bloquées en rotation par rapport à l'arbre et séparées par des entretoises (18).
Les canaux radiaux (15) peuvent être formés dans les entretoises (18) séparant les lames. Les canaux axiaux peuvent être réalises dans l'arbre ou dans les entretoises et les lames du broyeur. Il est par exemple envisageable d'utiliser un axe cannelé ou bien encore un axe creux doté de perçages radiaux.

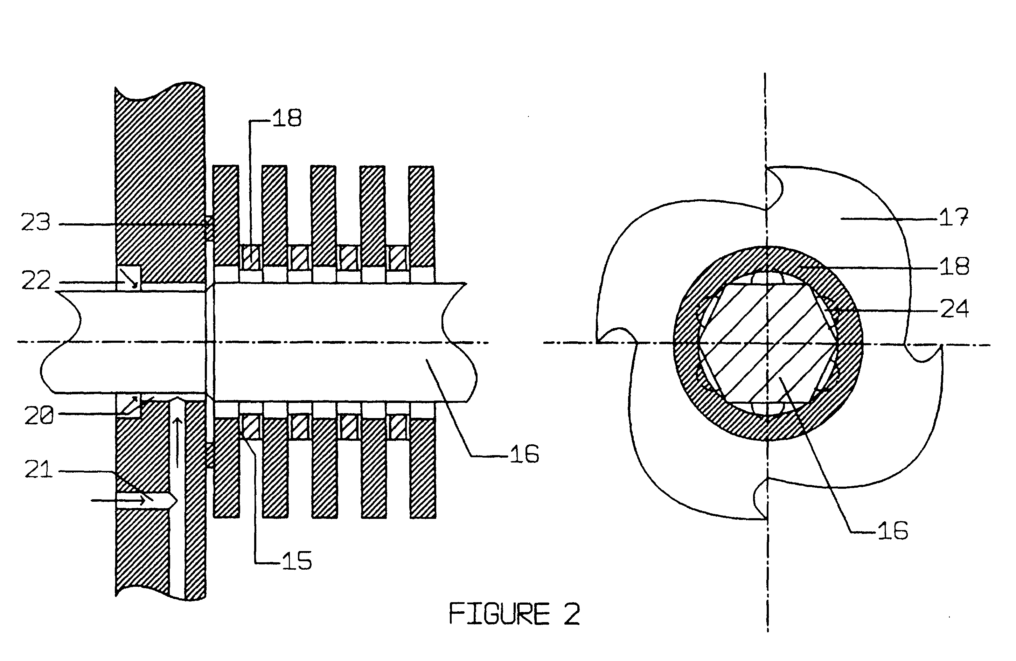

Selon le mode de réalisation représenté sur la figure 2, l'arbre (16) est hexagonal et les lames (17) sont montées sur cet arbre et séparées par des entretoises (18).

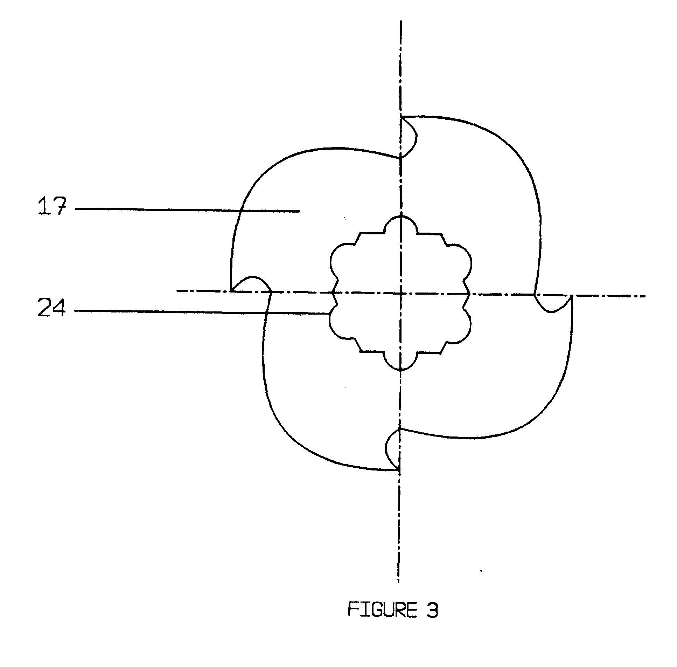

Comme on peut le voir sur la figure 3, l'alésage central des lames est hexagonal de manière à s'assembler sur l'arbre et à bloquer la rotation desdites lames par rapport à l'arbre. Chacune des faces de l'hexagone est dotée d'une encoche (24) pour permettre le passage de la vapeur au travers de la lame. La partie tranchante des lames est de type connu comportant plusieurs dents.

Les entretoises (18) sont de forme annulaire de diamètre intérieur correspondant au diamètre maximal de l'arbre hexagonal. Le fluide peut donc librement circuler dans l'espace formé entre chaque pan de l'arbre et la surface interne de l'entretoise. Ces espaces en combinaison avec les encoches des lames permettent la formation des canaux axiaux. Chacune des surfaces latérales des entretoises est pourvue de plusieurs encoches radiales (19) formant les canaux radiaux pour le passage du fluide.

Tel que représenté sur la figure 2, la vapeur représentée par des flèches est envoyée au travers d'un conduit (20) réalisé dans la paroi de l'enceinte de broyage. Ce conduit débouche dans une chambre annulaire (21) formée entre l'arbre du broyeur et un alésage réalisé dans la paroi de l'enceinte de broyage. Cette chambre est isolée de l'extérieur par un joint (22) mais communique vers l'intérieur de l'enceinte de broyage. L'espace entre la première lame du broyeur et la paroi de l'enceinte de broyage est isolé par un joint (23). La vapeur communique donc d'une lame vers l'autre au travers des encoches (24) et nettoie la surface des lames en s'échappant par les encoches radiales (19) des entretoises. La taille des encoches (19) peut varier sur la longueur de l'arbre pour compenser les pertes de charges. Ainsi, la taille des encoches peut augmenter en s'éloignant de l'orifice d'admission de vapeur (20) de manière à ce que la pression de soufflage sur chaque lame soit la même.

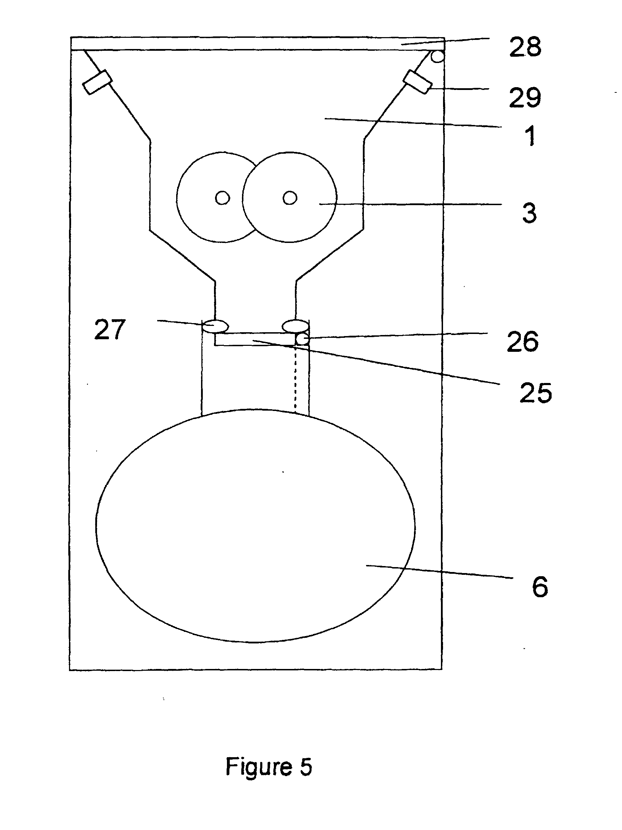

La figure 5 représente un autre mode de réalisation de l'invention dans lequel la vanne à tiroir est remplacée par un clapet articulé (25) selon un axe horizontal (26). L'autoclave est donc disposée à l'aplomb du broyeur. Le clapet (25) isole l'autoclave (6) de l'enceinte de broyage (12). Un joint torique (27) est disposé au dessus du clapet (25) de manière à ce que lorsque l'autoclave est sous pression, le clapet vienne comprimer le joint et assurer une parfaite étanchéité. L'orifice d'alimentation des déchets (1) peut également être constitué d'une trémie fermée par un couvercle (28). Des buses (29) peuvent être disposées régulièrement au sommet de la trémie de manière à faire couler un liquide bactéricide sur les parois de celle-ci. Selon un autre perfectionnement de l'invention, le plateau de pesage (5) des déchets peut être remplacé par une temporisation agissant sur le broyeur. En connaissant le débit du broyeur on peut déterminer le temps de fonctionnement du broyeur nécessaire au remplissage de l'autoclave.

Afin de faciliter son utilisation par de nombreuses professions médicales, le dispositif peut se présenter sous des dimensions réduites de l'ordre par exemple de 1,30m de hauteur par 1m de largeur et 1m de profondeur.

## Revendications

1. Procédé de broyage et de désinfection des déchets médicaux comportant une étape de broyage et une étape de stérilisation des déchets, **caractérisé en ce qu'**il comporte une étape consistant à projeter au moins un fluide directement sur les lames du broyeur, ledit fluide étant projeté dans une direction parallèle à la surface de chaque lame, depuis le centre de la lame vers l'extérieur afin d'éliminer et de décontaminer tous les résidus de déchets que lesdites lames comportent.

2. Procédé de broyage et de désinfection selon la revendication 1 **caractérisé en ce qu'**au moins un fluide projeté est de la vapeur d'eau.

3. Procédé de broyage et de désinfection selon la revendication 1 **caractérisé en ce qu'**au moins un fluide projeté est un liquide bactéricide.

4. Procédé de broyage et de désinfection selon la revendication 1 ou la revendication 2 **caractérisé en ce qu'**il comporte une étape consistant à remplir l'enceinte de broyage d'un liquide bactéricide puis à la vidanger.

5. Procédé de broyage et de désinfection selon la revendication 4 **caractérisé en ce qu'**il comporte une étape consistant à stocker une partie du liquide bactéricide.

6. Procédé de broyage et de désinfection selon la revendication 5 **caractérisé en ce que** le liquide bactéricide stocké est projeté sur les lames du broyeur.

7. Procédé de broyage et de désinfection selon la revendication 6 **caractérisé en ce que** la projection de liquide bactéricide est suivie d'une projection de vapeur d'eau.

8. Procédé de broyage et de stérilisation des déchets selon l'une des revendications précédentes **caractérisé en ce qu'**il consiste à broyer les déchets, les stériliser, les évacuer, à remplir l'enceinte de broyage d'un liquide bactéricide, la vidanger puis projeter au moins un fluide sur les lames du broyeur.

9. Dispositif de broyage et de désinfection de déchets médicaux comportant un dispositif de broyage du type à couteaux rotatifs comportant des moyens spécifiques de projection d'au moins un fluide de nettoyage sur les lames dudit broyeur , **caractérisé par** des canaux réalisés au niveau de chaque axe de rotation du broyeur, le fluide de nettoyage étant projeté au travers de ces canaux.

10. Dispositif selon la revendication 9 **caractérisé en ce que** la projection de fluide est effectuée au travers d'au moins un canal axial parallèle à l'axe de rotation auquel sont reliés un ou plusieurs canaux radiaux débouchant vers l'extérieur de manière à projeter le fluide sur la surface de chaque lame.

11. Dispositif selon l'une des revendications 9 ou 10 **caractérisé en ce que** chaque axe de broyage est constitué d'un arbre (16) et de lames (17) bloquées en rotation par rapport à l'arbre, lesdites lames étant séparées entre elles par des entretoises (18).

12. Dispositif selon la revendication 11 **caractérisé en ce que** les canaux radiaux sont constitués par des encoches radiales (19) formées sur au moins une des faces latérales de chaque entretoise.

13. Dispositif selon la revendication 11 ou la revendication 12 **caractérisée en ce que** l'arbre (16) est hexagonal, l'alésage central des lames étant également hexagonal et pourvu d'encoches (24) pour permettre le passage du fluide au travers de ladite lame, les entretoises (18) étant de forme annulaire de diamètre intérieur correspondant au diamètre maximal dudit arbre hexagonal, les canaux axiaux étant donc chacun constitués par l'espace formé entre chaque pan dudit arbre hexagonal et la surface interne de l'entretoise en combinaison avec les encoches (24) des lames.

14. Dispositif selon l'une des revendications 9 à 13 **caractérisé en ce qu'**il comporte une enceinte de broyage pourvue de joints étanches.

15. Dispositif selon la revendication 14 **caractérisé en ce qu'**il comporte des moyens de remplissage et de vidange de l'enceinte de broyage par un liquide bactéricide.

16. Dispositif selon la revendication 14 ou la revendication 15 **caractérisé en ce qu'**il comporte des moyens de stockage d'une partie du liquide bactéricide ayant rempli l'enceinte de broyage, lesdits moyens de stockage étant constitués par les canaux de projection de fluide.

17. Dispositif selon l'une des revendications 9 à 16 **caractérisé en ce qu'**il est composé:
- d'un orifice d'alimentation (1) des déchets,
- d'une gaine souple (2) enfilée sur l'orifice d'alimentation des déchets et dont l'extrémité supérieure est introduite dans l'orifice de manière à recouvrir la paroi interne dudit orifice,
- d'un dispositif de broyage (3) contenu dans une enceinte de broyage (12)
- d'une vanne à tiroir (4)
- d'un plateau de pesage (5)
- d'un autoclave (6) de désinfection des déchets
- d'un plateau de pressage des déchets (7)
- d'un orifice d'extraction des déchets désinfectés.

18. Dispositif selon l'une des revendication 9 à 16 **caractérisé en ce qu'**il est composé :
- d'un orifice (1) d'alimentation des déchets fermé par un couvercle (28)
- d'un dispositif de broyage (3) contenu dans une enceinte de broyage (12), le temps de fonctionnement du broyeur étant contrôlé par une temporisation,
- d'un autoclave (6) de désinfection des déchets
- d'un clapet articulé (25) isolant hermétiquement l'autoclave (6)
- d'un orifice d'extraction des déchets désinfectés.

19. Dispositif selon la revendication 10 **caractérisé en ce que** la dimension des canaux radiaux augmente en s'éloignant de l'orifice d'admission de vapeur (20) pour compenser les pertes de charge.

## Patentansprüche

1. Mahl- und Desinfektionsverfahren für medizinische Abfälle, die in zwei separaten Schritten gemahlen und sterilisiert werden, **gekennzeichnet dadurch, dass** es einen Schritt enthält, in dem mindestens eine Flüssigkeit direkt auf die Messer des Mahlwerks gespritzt wird, so dass diese Flüssigkeit in eine zur Oberfläche der Messer parallel liegende Richtung gespritzt wird, d.h. vom Mittelpunkt des Messers nach außen gerichtet, um alle an diesen Messern haftenden Abfallreste zu beseitigen und zu dekontaminieren.

2. Mahl- und Desinfektionsverfahren gemäß Anspruch 1, **gekennzeichnet dadurch, dass** mindestens eines der aufgespritzten Fluide Wasserdampf ist.

3. Mahl- und Desinfektionsverfahren gemäß Anspruch 1, **gekennzeichnet dadurch, dass** mindestens eines der aufgespritzten Fluide eine bakterizide Flüssigkeit ist.

4. Mahl- und Desinfektionsverfahren gemäß Anspruch 1 oder 2, **gekennzeichnet dadurch, dass** es einen Schritt aufweist, bei dem die Mahlkammer mit einer bakteriziden Flüssigkeit gefüllt und anschließend entleert wird.

5. Mahl- und Desinfektionsverfahren gemäß Anspruch 4, **gekennzeichnet dadurch, dass** es einen Schritt aufweist, bei dem ein Teil der bakteriziden Flüssigkeit gelagert wird.

6. Mahl- und Desinfektionsverfahren gemäß Anspruch 5, **gekennzeichnet dadurch, dass** die gelagerte Flüssigkeit auf die Messer des Mahlwerks gespritzt wird.

7. Mahl- und Desinfektionsverfahren gemäß Anspruch 6, **gekennzeichnet dadurch, dass** auf das Aufspritzen der bakteriziden Flüssigkeit ein Aufspritzen von Wasserdampf folgt.

8. Mahl- und Sterilisierungsverfahren für Abfälle gemäß einem der vorstehenden Ansprüche, **gekennzeichnet dadurch, dass** die Abfälle gemahlen, sterilisiert, ausgegeben, die Mahlkammer mit einer bakteriziden Flüssigkeit gefüllt, entleert und mindestens ein Fluid auf die Messer des Mahlwerks gespritzt wird.

9. Mahl- und Desinfektionsverfahren für medizinische Abfälle mit einem Mahlwerk mit rotierenden Messern, das spezifische Mittel zum Aufspritzen von mindestens einer Reinigungsflüssigkeit auf die Messer des genannten Mahlwerks besitzt, **gekennzeichnet dadurch, dass** Kanäle auf Ebene jeder Drehachse des Mahlwerks angeordnet sind und dass die Reinigungsflüssigkeit durch diese Kanäle hindurch verspritzt wird.

10. Vorrichtung gemäß Anspruch 9, **gekennzeichnet dadurch, dass** das Verspritzen des Fluids mit Hilfe mindestens eines, zur Rotationsachse parallel liegenden Kanals erfolgt, der mit einem oder mehreren radialen Kanälen verbunden ist, die so nach außen münden, dass das Fluid auf die Oberfläche der einzelnen Messer gespritzt wird.

11. Vorrichtung gemäß einem der Ansprüche 9 oder 10, **gekennzeichnet dadurch, dass** jede Mahlachse aus einer Welle (16) und auf der Welle drehfest sitzenden Messern (17) besteht und die Messer durch Distanzstücke (18) voneinander getrennt sind.

12. Vorrichtung gemäß Anspruch 11, **gekennzeichnet dadurch, dass** die radialen Kanäle durch radiale Nuten (19) auf mindestens einer der seitlichen Flächen jedes Distanzstücks ausgebildet sind.

13. Vorrichtung gemäß Anspruch 11 oder 12, **gekennzeichnet dadurch, dass** die Welle (16) hexagonal ist, die zentrale Bohrung der Messer ebenfalls hexagonal ist und Nuten (24) besitzt, um das Durchtreten der Flüssigkeit durch das Messer zu ermöglichen, wobei der Innendurchmesser der ringförmigen Distanzstücke (18) dem maximalen Außendurchmesser der genannten hexagonalen Welle entspricht, so dass die axialen Kanäle aus dem Raum zwischen einer Fläche der Sechskantwelle und der inneren Oberfläche des Distanzstückes in Kombination mit den Nuten (24) der Messer gebildet werden.

14. Vorrichtung gemäß einem der Ansprüche 9 bis 13, **gekennzeichnet dadurch, dass** sie eine Mahlkammer besitzt, die mit Dichtungen versehen ist.

15. Vorrichtung gemäß Anspruch 14, **gekennzeichnet dadurch, dass** sie Mittel zum Befüllen und Entleeren der Mahlkammer mit einer bakteriziden Flüssigkeit besitzt.

16. Vorrichtung gemäß Anspruch 14 oder 15, **gekennzeichnet dadurch, dass** sie Mittel zur Lagerung eines Teils der bakteriziden Flüssigkeit besitzt, mit dem die Mahlkammer gefüllt worden war und dass diese Lagerungsmittel aus den Spritzkanälen der Flüssigkeit bestehen.

17. Vorrichtung gemäß einem der Ansprüche 9 bis 16, **gekennzeichnet dadurch, dass** sie besteht aus:
• einer Einfüllöffnung (1 ) für die Abfälle,
• einem Schlauch (2), der auf die Einfüllöffnung der Abfälle gesteckt wird und dessen oberes Ende so in die Öffnung eingeführt wird, dass er die Innenwand der genannten Öffnung bedeckt,
• einem Mahlwerk (3) in einer Mahlkammer (12),
• einem Absperrschieber (4),
• einer Waage (5)
• einem Autoklaven (6) zur Desinfektion der Abfälle,
• einer Platte (7) zum Pressen der Abfälle,
• einer Ausgabeöffnung der desinfizierten Abfälle.

18. Vorrichtung gemäß einem der Ansprüche 9 bis 16, **gekennzeichnet dadurch, dass** sie besteht aus:
• einer Öffnung (1) zum Einfüllen der Abfälle, die mit einem Deckel (28) verschlossen ist,
• einem Mahlwerk (3) in einer Mahlkammer (12), dessen Betrieb von einem Zeitgeber gesteuert wird,
• einem Autoklaven (6) zur Desinfektion der Abfälle,
• einer drehbar gelagerten Klappe (25) zum dichten Verschließen des Autoklaven (6),
• einer Ausgabeöffnung der desinfizierten Abfälle.

19. Vorrichtung gemäß Anspruch 10, **gekennzeichnet dadurch, dass** die Abmessungen der radialen Kanäle von der Eintrittsöffnung des Dampfes (20) nach außen zunimmt, um den Druckverlust zu kompensieren.

## Claims

1. Process for shredding and disinfecting medical waste comprising a shredding stage and a sterilization stage, **characterized in that** it includes a stage that consists in spraying at least one fluid directly onto the blades of the shredder, the aforementioned fluid being sprayed in a direction parallel to the surface of each blade, from the centre of the blade towards the outside in order to eliminate and decontaminate all the waste residues on the aforementioned blades.

2. Shredding and disinfection process according to claim 1 **characterized in that** at least one of the sprayed fluids is steam.

3. Shredding and disinfection process according to claim 1 **characterized in that** at least one of the sprayed fluids is a bactericidal liquid.

4. Shredding and disinfection process according to claim 1 or claim 2 **characterized in that** it includes a stage that consists in filling the shredding chamber with a bactericidal liquid and then draining it.

5. Shredding and disinfection process according to claim 4 **characterized in that** it includes a stage that consists in storing part of the bactericidal liquid.

6. Shredding and disinfection process according to claim 5 **characterized in that** the stored bactericidal liquid is sprayed onto the blades of the shredder.

7. Shredding and disinfection process according to claim 6 **characterized in that** spraying of bactericidal liquid is followed by spraying the steam.

8. Process for shredding and sterilization of waste according to one of the preceding claims **characterized in that** it consists in shredding, sterilizing, evacuating waste, in filling the shredding chamber with a bactericidal liquid, draining it and then spraying at least one fluid on the blades of the shredder.

9. Device for shredding and disinfection of medical waste comprising a rotary knife type shredding device with specific means for spraying at least one cleaning fluid on the blades of the aforesaid shredder, **characterized by** channels created at each shredder axis of rotation, the cleaning fluid being sprayed through these channels.

10. Device according to claim 9 **characterized in that** the fluid is sprayed through at least one axial channel parallel to the axis of rotation to which are connected one or more radial channels emerging on the outside so as to spray the fluid onto the surface of each blade.

11. Device according to one of claims 9 or 10 **characterized in that** each shredding axis consists of a shaft (16) and blades (17) blocked in rotation relative to the shaft, the aforementioned blades being separated between them by spacers (18).

12. Device according to claim 11 **characterized in that** the radial channels consist of radial notches (19) formed on at least one of the sides of each spacer.

13. Device according to claim 11 or claim 12 **characterized in that** shaft (16) is hexagonal, the central bore of the blades also being hexagonal and equipped with notches (24) to allow the passage of the fluid through the aforementioned blade, spacers (18) being of annular shape with inner diameter corresponding to the maximum diameter of the aforesaid hexagonal shaft, each axial channels thus consisting of the space formed between each flat of the aforesaid hexagonal shaft and the inner surface of the spacer in combination with notches (24) of the blades.

14. Device according to one of claims 9 to 13 **characterized in that** it comprises a shredding chamber equipped with seals.

15. Device according to claim 14 **characterized in that** it includes means for filling and draining the shredding chamber with a bactericidal liquid.

16. Device according to claim 14 or claim 15 **characterized in that** it includes means for storage of part of the bactericidal liquid that has filled the shredding chamber, the aforementioned storage means consisting of the spray liquid channels.

17. Device according to one of claims 9 to 16 **characterized in that** it consists of:
- a waste feed orifice (1),
- a flexible sheath (2) threaded on the waste feed orifice and whose upper end is inserted into the orifice so as to cover the inner wall of the aforesaid orifice,
- a shredding device (3) contained in the shredding chamber (12),
- a slide valve (4),
- a weighing pan (5),
- an waste disinfection autoclave (6),
- a waste press plate (7),
- a disinfected waste extraction orifice.

18. Device according to one of claims 9 to 16 **characterized in that** it consists of:
- an orifice (1) feeding the waste enclosed by a cover (28),
- a shredding device (3) contained in a shredding chamber (12), the shredder operating time being controlled by a time delay mechanism,
- a waste disinfection autoclave (6),
- a hinged flap valve (25) sealing the autoclave (6),
- an orifice for extracting the disinfected waste.

19. Device according to claim 10 **characterized in that** the dimension of the radial channels increases when moving away from the steam intake port (20) to compensate for pressure drops.
